# EUROPÄISCHE PATENTSCHRIFT

(11) **EP 0 347 874 B1**
(45) Veröffentlichungstag und Bekanntmachung des Hinweises auf die Patenterteilung: **02.03.1994**
(21) Anmeldenummer: 89111280.7
(22) Anmeldetag: 21.06.1989
(51) Int. Cl.: A61B 17/58

(54) **Vorrichtung zum Verbinden eines, insbesondere im Bereich des Oberschenkelhalses, gebrochenen Knochens**
Device for the fixation of a broken bone, in particular in the region of the femoral neck
Dispositif pour fixer un os fracturé, en particulier dans la région du col du fémur

(30) Priorität: 24.06.1988 DE 8808123 U
(43) Veröffentlichungstag der Anmeldung: 27.12.1989
(73) Patentinhaber: Dunsch-Herzberg, Renate, D-22880 Wedel (DE); Voss, Gudrun, D-25491 Hetlingen (DE)
(72) Erfinder: Herzberg, Wolfgang, Dr.med., D-2000 Wedel/Holstein (DE)
(74) Vertreter: Richter, Werdermann & Gerbaulet

(56) Entgegenhaltungen:
- DE-A- 3 006 518
- DE-U-82 132 283
- FR-A- 2 149 943
- US-A- 4 438 762
- US-A- 4 628 923

## Beschreibung

Die Erfindung betrifft eine Vorrichtung zum Verbinden eines, insbesondere im Bereich des Oberschenkelhalses,gebrochenen Knochens, bestehend aus einer mit dem Knochen durch eine Schraubverbindung verbindbaren, parallel zur Knockenlängsachse verlaufenden Knochenbefestigungslasche mit Befestigungsdurchbrechungen, mit einer abgewinkelten Hülse zur Aufnahme einer Knochenschraube oder eines Knochennagels, sowie einer Trochanterabstützplatte, wobei der Steg eines laschenförmigen Abschnittes der Trochanterabstützplatte einendseitig in einen sich erweiternden plattenförmigen Abschnitt als Trochanterabstützfläche übergeht und beidseitig je einen sich in Steghängsrichtung erstreckenden Versteifungsschenkel aufweist, der sich über eine Teillänge des Steges oder über die gesamte Länge des Steges bis in einen Teil oder bis in den Endbereich des plattenförmigen Abschnittes erstreckt und sich zu dem Endbereich hin konisch verjüngt, wobei der plattenförmige Abschnitt als Trochanterabstützfläche nach cranial über die Knochenbefestigungslasche hinausragt.

Eine derartige Vorrichtung zum Verbinden eines, insbesondere im Bereich des Oberschenkelhalses, gebrochenen Knochens ist durch die DE-U-82 13 228.3 bekannt. Bei dem dort beschriebenen Implantat zur Versorgung von trochanteren Oberschenkelfrakturen sind die Trochanterabstützplatten unlösbar mit der Knochenbefestigungslasche verbunden; hier ist eine einstückige Ausgestaltung gegeben, wodurch keine ausreichende Anpassungsfähigkeit an unterschiedlich anatomische Gegebenheiten gegeben ist. Durch diese einstückige Ausgestaltung ist es nicht möglich, Teile unterschiedlicher Ausgestaltung und unterschiedlicher Abmessungen bausatzsystemartig miteinander so zu kombinieren, daß die Verbindungsvorrichtung den jeweiligen anatomischen Gegebenheiten angepaßt werden kann.

Für Osteosynthese pertrochantärer Frakturen, d.h. zur stabilen Retention von Knochenfragmenten bei Quer- oder kurzer Schrägfraktur ist es bekannt, die Frakturen des Oberschenkelhalses durch dynamische Laschenschrauben zu versorgen. Derartige Laschenschrauben bestehen aus einer Knochenschraube, die vom Trochanter,d.h. vom großen Rollhügel, der lateral am proximalen Femurschaft sitzt, in das proximale Fragment eingetrieben oder eingeschraubt wird und aus einer Knochenplatte, die durch Schrauben am distalen Fragment fixiert wird. Zur Anwendung hierbei gelangen sogenannte Pohl'sche Laschenschrauben, die aus der Knochenplatte und einer in einem Winkel zu dieser an dieser angeformten Hülse zur Aufnahme der Knochenschraube oder eines Knochennagels bestehen, wobei die Knochenplatte mittels Schraubverbindungen parallel zur Knochenlängsachse verlaufend und am Knochen anliegend an diesem befestigt wird.

Des weiteren ist es bekannt, an der Knochenanlagenplatte derartiger Laschenschrauben eine Trochanterabstützplatte anzuformen, die nach cranial, also kopfwärts über die eigentliche Befestigungslasche bzw. Knochenanlageplatte hinausragt und eine dem Trochanter in etwa angepaßte Formgebung aufweist, und nach Befestigung der Laschenschraube am Knochen am Trochanter anliegt und so den Trochanter abstützt. Es hat sich nämlich gezeigt, daß, wenn der Schenkelhalsbruch in den Trochanter hineinreicht bzw. bis unter den Trochanter hinabreicht, dann die sonst so ideale Versorgungstechnik die Grenzen ihrer Leistungsfähigkeit erreicht hat. Um die Leistungsfähigkeit der Laschenschraube bei derartigen Bruchformen zu verbessern, sind die bekannten Laschenschrauben daher mit einer Trochanterabstützplatte versehen. Wesentlich ist jedoch, daß eine mit einer Trochanterabstützplatte versehene Laschenschraube ein einstückiges Implantat bildet, was voraussetzt, daß dem Chirurgen ein umfangreiches Sortiment neben dem bereits vorhandenen Implantatsortiment zur Verfügung stehen muß.

Nach der DE-A-20 30 249 ist eine Vorrichtung für chirurgische Zwecke zur Verbindung von einem, insbesondere im Bereich des Oberschenkelhalses, gebrochenen Knochen bekannt, der über in diesen eingreifende Schrauben mit einer Schiene verbunden ist, an der an einem Ende ein in einem Winkel zu dem Knochen einstellbares Verbindungselement angeordnet ist, das an einem kreisbogenförmig ausgebildeten Halter angebracht ist, der in einer Kreiskalotte in der Schiene drehbar und feststellbar angeordnet ist, wodurch die Möglichkeit gegeben ist, verschiedenartig ausgebildete Schienen zu verwenden, wobei jede Schiene lediglich an ihrem einen Ende mit einer Kreiskalotte ausgestaltet ist, in der der Halter drehbar ist. Dadurch, daß der Halter für das Verbindungselement an der Schiene befestigt ist, bedarf es in zahlreichen Fällen nur der zusätzlichen Halter und keiner eigens konstruierten Schiene, was immer dann von Vorteil ist, wenn zu einem Oberschenkelbruch noch ein Oberschenkelhalsbruch hinzu kommt, der dann den Einsatz eines Verbindungselementes fordert, während hingegen es bei einem Bruch eines Oberschenkelknochens nur einer Schiene bedarf, wobei je nach Größe des Oberschenkels und nach der Art des Bruches verschiedene Schienenformen verwendet werden. Dadurch, daß der Halter jedoch drehbar an der Schiene befestigt ist und die Verbindung vermittels einer Schraubverbindung erfolgt, ist nicht ausschließbar, daß sich der Halter mit dem Verbindungselement zur Schiene verdreht oder daß bereits beim Anlegen und Befestigen der Gesamtvorrichtung eine Verschiebung des Halters zur Schiene erfolgt, was zur Folge hat, daß bei einem Oberschenkelhalsbruch sich der Oberschenkelhals im Bereich der Bruchfläche zum Oberschenkelknochen verschiebt. Hinzu kommt noch, daß eine Trochanterabstützfläche überhaupt nicht vorhanden ist. Denn der vorgesehene und an der Schiene befestigte Halter liegt bei montierter Verbindung nicht am Trochanter an, sondern steht unter Ausbildung eines Zwischenraumes von der Trochanterfläche ab; auch eine Formgebung des Halters in der Art, daß der Trochanter abschnittsweise umgriffen wird, ist nicht vorgesehen.

Die EP-A-0 046 773 betrifft eine Vorrichtung zum Fixieren des Oberschenkelhalses und des großen Rollhügels am Schaft eines Oberschenkelknochens eines Menschens. Diese Vorrichtung ist gekennzeichnet durch eine Winkelplatte, deren als Klingenteil ausgebildeter Schenkel axial in den Hals eingeschlagen wird und deren als Schaftabschnitt ausgebildeter andere Schenkel am Schaft fixiert, und durch eine Krallenplatte, die am Schaftabschnitt der Winkelplatte und/oder am Schaft befestigt wird und den Rollhügel am Hals fixiert. Zwecks Drehung des Oberschenkelhalses um 90°, um die zerstörte Stelle am Oberschenkelhals aus dem Belastungsbereich zu entfernen und eine unzerstörte Stelle des Gelenkkopfes in den Belastungsbereich zu bringen, muß der Klingenteil der Vorrichtung in den Hals eingeschlagen werden. Weist der Femurkopf eine Vielzahl von Bruchflächen auf und ist z.B. ein erheblicher Bereich des Trochanter major abgegriffen, dann würde das Einschlagen des Klingenteils zu einer weiteren Beschädigung des Femurkopfes führen. Hinzu kommt, daß in zahlreichen Fällen ein Einschlagen von Teilen in den Oberschenkelhals bzw. in den Femurkopf überhaupt nicht möglich ist, so daß diese Vorrichtung nicht bei allen Oberschenkelhalsbrüchen einsetzbar ist. Diese Vorrichtung ist zweiteilig ausgebildet und besteht aus einer Krallenplatte und einer Winkelplatte, wobei die Krallenplatte auf dem Schaftabschnitt der Winkelplatte in Schaftlängsrichtung verschieblich ist; der Schaftabschnitt der Winkelplatte und die Krallenplatte müssen mittels Schrauben aufeinanderliegend am Schaft befestigt werden. Diese Vorrichtung ist ausschließlich ausgebildet für ein Fixieren des Oberschenkelhalses und des großen Rollhügels am Schaft eines Oberschenkelknochens. Es wird dabei davon ausgegangen, daß die Fixierung zur Heilung eines Bruches notwendig wird, und zwar z.B. eines solchen Doppelbruches, bei dem sich der Rollhügel vom Oberschenkelhals und der Oberschenkelhals vom Schaft gelöst haben, aber auch in anderen Fällen, wie z.B. in dem, bei dem der Gelenkkopf in seinem Belastungsbereich zerstört ist. Dann wird der Oberschenkelhals vom Schaft und vom Rollhügel getrennt und um 90° gedreht, derart, daß ein gesunder Bereich des Gelenkkopfes Belastungsbereich wird. Aus diesem Grunde muß diese Fixierungsvorrichtung einen Teil aufweisen, nämlich den Klingenteil, der in den Hals eingeschlagen wird, um den Oberschenkelhals nach Trennung vom Schaft und vom Rollhügel verdrehen zu können.

Ein Schenkelhalsimplantat mit einer Schenkelhalsplatte, welche zu ihrer Befestigung außen am Femur entlang ihrer Längsachse mehrere Aufnahmeöffnungen für Kortikalis-Schrauben aufweist und die an ihrem oberen Bereich mit einem sich stumpfwinklig in ihrer Längsachse erstreckenden, subtrochantär bis in den Schenkelhals und ggf. in den Femurkopf hineinreichend zu implantierenden Schaft versehen ist, ist ferner durch die DE-A-35 34 747 bekannt. Bei diesem einteilig ausgebildeten Schenkelhalsimplantat bildet sein Oberbereich einen etwa der Außenkontur des Trochanter major angepaßten bzw. anpaßbaren, einstückig angeschlossenen Fortsatz, der eine oder mehrere Aufnahmeöffnungen für sich pertrochantär bis in den Schenkelhals bzw. bis in den Femurkopf hineinreichend zu befestigende Spongiosa-Schrauben aufweist, wobei der Schaft außen glatt und kreiszylindrisch ausgebildet und mit einem endseitigen Außengewinde in einer Innengewindeaufnahme im oberen Bereich der Schenkelhalsplatte lösbar befestigt ist.

Ein derart ausgebildetes Schenkelhalsimplantat soll mit geringem operativen Aufwand unter Einhaltung des physiologischen Schenkelhalswinkels einsetzbar sein, und zwar auch bei instabilen pertrochantären Frakturen eine primäre postoperative Belastung gestattet, ohne daß die Gefahr eines mechanischen Zusammenbruches der vorgenommenen Osteosynthese besteht. Bei diesem Schenkelhalsimplantat geht es jedoch im wesentlichen um die Befestigung einer einstückig ausgebildeten Schenkelhalsplatte mit einem angeformten, plattenförmigen Abschnitt als Trochanterabstützfläche. Durch die einstückige Ausgestaltung der Schenkelhalsplatte ist keine Anpassungsfähigkeit an die verschiedensten anatomischen Gegebenheiten und keine Kombinierbarkeit mit anderem Implantatsortiment für per- und subtrochantäre Oberschenkelfrakturen möglich.

Die DE-A-10 46 827 beschreibt eine einteilige Verbindungsvorrichtung für gelenknahe Knochenbrüche, wobei diese Verbindungsvorrichtung aus einem einstückig ausgebildeten Formkörper besteht, der einen Abschnitt als Knochenbefestigungslasche und einen Abschnitt als Trochanterabstützfläche aufweist. Im Bereich der kreisbogenförmig verlaufend ausgebildeten Trochanterabstützfläche sind Durchbohrungen bzw. Durchbrechungen zum Anbringen von Schraubennägeln , Befestigungsschrauben u. dgl. vorgesehen, wobei der Formkörper der Verbindungseinrichtung mit einer Schraubennagelführungshülse versehen ist, die ein Innengewinde trägt, in das ein Außengewinde eingreift, welches im Schraubenkopfbereich des Schraubennagels ausgebildet ist.

Der Hüftnagel nach der DE-A-18 13 807 besteht aus einer Schaftplatte, die entlang der seitlichen Fläche des Oberschenkelknochenschaftes unterhalb des Rollhügels zur Anlage gebracht wird und mit einer Trochanteranlageplatte versehen ist sowie ferner einen Nagelteil aufweist, der einen Durchgang bzw. eine axiale Bohrung aufweist, die dazu dient, eine Druckvorrichtung aufzunehmen, die durch einen länglichen Schaft oder Stiel gekennzeichnet ist, der an seinem äußeren Ende mit einem Gewinde ausgebildet ist und der an seinem entgegengesetzten Ende schwenkbare Greifelemente trägt, so daß bei einem Anziehen des Hüftnagels der in seiner Bruchstelle auszurichtende Oberschenkelhalsteil gegen die am Oberschenkelknochen befestigte Schaftplatte gepreßt wird. Auch diese Verbindungsvorrichtung ist einstückigh ausgebildet.

Die US-A-2 443 363 beschreibt eine Schaftplatte zur Befestigung an einem Oberschenkelknochen, wobei die Schaftplatte aus einem etwa U-förmigen Profilkörper besteht und über einen ausziehbaren Abschnitt in seiner Länge veränderbar ist. Auch wenn diese Schaftplatte zweiteilig ausgebildet ist, so dient diese zweiteilige Ausgestaltung ausschließlich dazu, die Schaftplatte in ihrer Länge verändern zu können. In keiner Weise besteht jedoch die Schaftplatte nach dieser amerikanischen Druckschrift aus einer Knochenbefestigungslasche mit einer Knochenschraubenaufnahmenhülse und der eigentlichen Trochanterabstützplatte mit einer Durchbrechung zum Hindurchführen der Knochenschraubenaufnahmehülse der Knochenbefestigungslasche.

Der Erfindung liegt die Aufgabe zugrunde, eine Vorrichtung zum Verbinden eines, insbesondere im Bereich des Oberschenkelhalses, gebrochenen Knochens, und somit eine Laschenschraube für die Osteosynthese pertrochantärer Frakturen gemäß der eingangs beschriebenen Art zu schaffen, die zur Erzielung einer höheren Anpassungsfähigkeit an anatomische Gegebenheiten eine beliebige Kombinierbarkeit bei einem geringeren Sortiment mit dem vorhandenen Implantatsortiment eines Herstellers für per- und subtrochantäre Oberschenkelfrakturen ermöglicht.

Diese Aufgabe wird durch die im Patentanspruch 1 gekennzeichneten Merkmale gelöst.

Aufgrund einer derartigen Ausgestaltung besteht die erfindungsgemäße Vorrichtung aus zwei Teilen, nämlich der Knochenbefestigungslasche mit der in einem Winkel zu der Knochenbefestigungslasche stehenden Hülse zur Aufnahme der Knochenschraube oder eines Knochennagels und dem Profilkörper mit einem der Knochenbefestigungslasche der Vorrichtung bzw. der Laschenschraube etwa entsprechenden laschenförmigen Abschnitt mit einem sich erweiternden, plattenförmigen Abschnitt als Trochanterabstützfläche, die die eigentliche Trochanterabstützplatte darstellt, wobei der Profilkörper im Bereich seines laschenartig ausgebildeten Abschnittes so ausgestaltet ist, daß dieser laschenförmige Abschnitt bei der Anbringung der Vorrichtung die Knochenbefestigungslasche übergreift, d.h., die Knochenbefestigungslasche wird in den laschenartigen Abschnitt des Profilkörpers eingelegt, so daß der plattenförmige Abschnitt als Trochanterabstützfläche cranial über die Knochenbefestigungslasche hinausragt und so den Trochanter abstützen kann, wobei dieser plattenförmige Abschnitt des Profilkörpers der Formgebung des Trochanters in etwa entspricht. Für einen sicheren Halt der Knochenbefestigungslasche an dem Profilkörper mit seiner Trochanterabstützplatte weist der laschenartig ausgebildete Abschnitt des Profilkörpers einen etwa U-förmigen Querschnitt auf, wobei der die beiden Profilschenkel miteinander verbindende Steg vorzugsweise eine den Profilkörperschenkeln zugekehrte, kreisbogenförmige Auswölbung aufweist, so daß die auf dem Knochen anliegende Fläche des Profilkörpers in etwa der Formgebung des Knochens angepaßt ist.

Durch diese zweiteilige Ausgestaltung ist es möglich, auf der Basis eines Bausatz-Systems das vorhandene Implantatsortiment des Herstellers und wie dieses dem Chirurgen zur Verfügung steht zu ergänzen. Durch eine beliebige Kombinierbarkeit kann bei geringerem Sortiment eine höhere Anpassungsfähigkeit an anatomische Gegebenheiten erzielt werden.

Des weiteren erbringt die zweiteilige Ausgestaltung der Vorrichtung wirtschaftliche Vorteile, denn das Implantatsortiment kann auf nur wenige Teile beschränkt werden, da die Möglichkeit besteht, Laschenschrauben bzw. Vorrichtungen verschiedener Abmessungen mit der Trochanterabstützplatte, bestehend aus dem Profilkörper mit dem laschenförmigen Abschnitt und dem erweiterten plattenförmigen Kopfabschnitt, miteinander zu kombinieren. Die Leistungsfähigkeit einer derart ausgebildeten Vorrichtung mit gesondert ausgebildeter Trochanterabstützplatte wird auch bei komplizierten Bruchformen wesentlich verbessert. Hinzu kommt, daß die Vorrichtung eine hohe Leistungsfähigkeit aufweist, denn auch komplizierte Bruchformen sind erfaßbar.

Vorteilhafte Ausgestaltungen der Erfindung sind in den Unteransprüchen gekennzeichnet, wobei besonders vorteilhaft die Ausgestaltung nach Anspruch 5 ist, nach der die Trochanterabstützplatte mit Einrichtungen versehen ist, die die Fixierung von Cerclage-Draht ermöglicht. Mit einer derartigen Cerclage kann der zersprengte Trochanter major korbartig an den löffelartigen Abschnitt der Abstützplatte herangebunden werden. Da der löffelartige Abschnitt der Abstützplatte anatomiegerechte Maße und Gestalt aufweist, dient dieser zusätzlich sowohl zur Stützung als auch als Formgeber, der die Ausheilung in eine anatomiegerechte Gestalt zurückführt. Eingesetzt wird die Vorrichtung zusammen mit einer Cerclage, wenn im Bereich des Trochanter major eine derartige Trümmerung vorliegt, daß die knöcherne Substanz nicht mehr schraubfähig ist.

Ausführungsbeispiele der Erfindung werden nachstehend anhand der Zeichnung näher erläutert. Es zeigen
Fig. 1 in einer Seitenansicht die Trochanterabstützplatte in Kombination mit der Knochenbefestigungslasche,
Fig. 2 in einer Ansicht von unten auf die Trochanterabstützplatte,
Fig. 3 eine schaubildliche Ansicht der Trochanterabstützplatte,
Fig. 4 einen waagerechten Schnitt gemäß Linie IV-IV in Fig.3,
Fig. 5 in einer Draufsicht ein plattenförmiges Verlängerungselement kurzer Länge für die Trochanterabstützplatte,
Fig. 6 in einer Draufsicht ein plattenförmiges Verlängerungselement größerer Länge für die Trochanterabstützplatte;
Fig. 7 einen waagerechten Schnitt gemäß Linie VII-VII in Fig. 6,
Fig. 8 in einer Draufsicht eine weitere Ausführungsform der Trochanterabstützplatte,
Fig. 9 in einem senkrechten Längsschnitt die Trochanterabstützplatte gem. Fig.8,
Fig.10 einen waagerechten Schnitt gemäß Linie X-X in Fig.9,
Fig.11 in einer Seitenansicht die Trochanterabstützplatte,
Fig. 12 in einer Ansicht von unten eine Trochanterabstützplatte mit Stabilisatoren,
Fig. 13 eine Seitenansicht der Trochanterabstützplatte gem. Fig. 12,
Fig. 14 einen waagerechten Schnitt gem. Linie XIV-XIV in Fig. 12,
Fig. 15 in einer Vorderansicht die Trochanterabstützplatte,
Fig. 16 teils in einer Seitenansicht, teils in einem senkrechten Schnitt die Trochanterabstützplatte gem. Fig.12 in Kombination mit der Knochenbefestigungslasche,
Fig. 17 in einer schaubildlichen Rückansicht eine Trochanterabstützplatte mit Einrichtungen zum Anbringen von Cerclage-Drähten, und
Fig. 18 in einer Seitenansicht die Trochanterabstützplatte gem. Fig. 17.

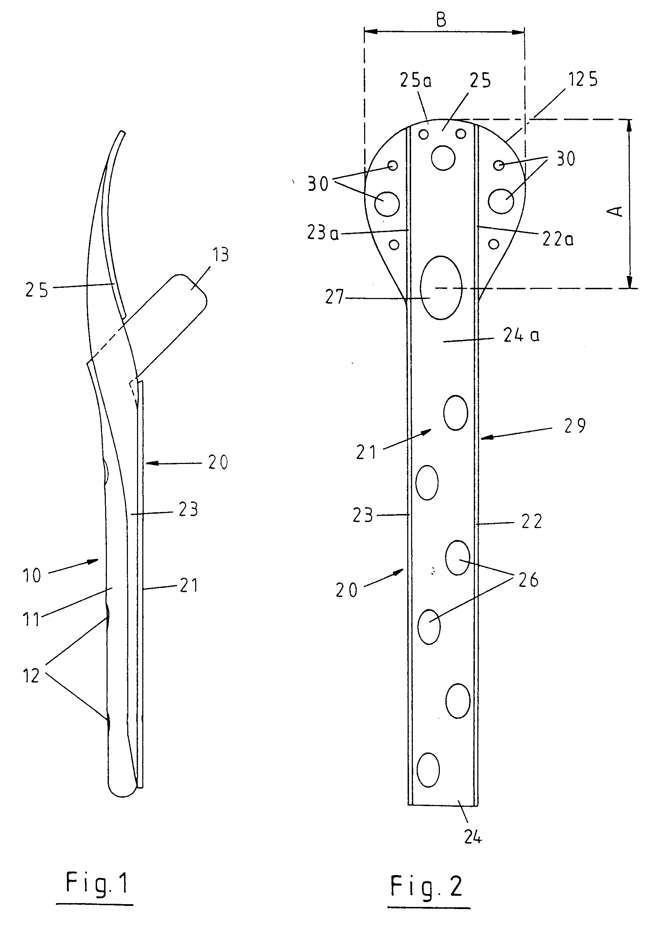

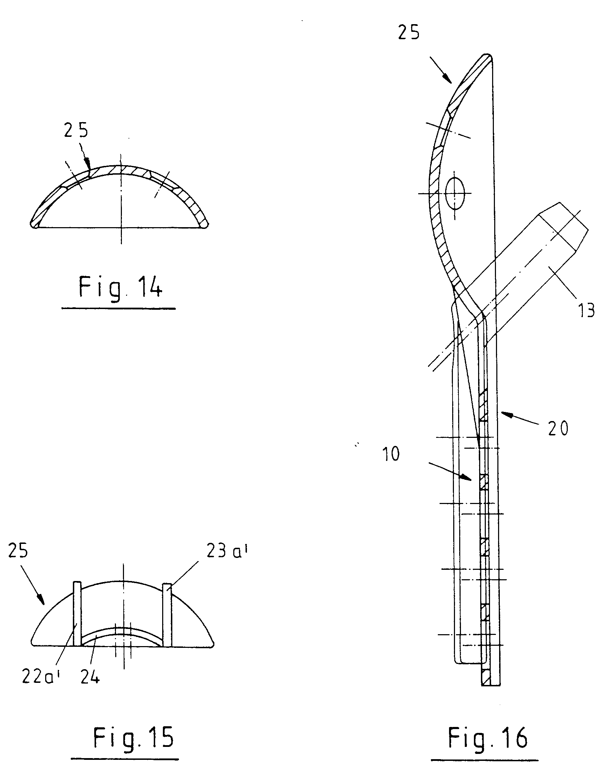

Gemäß Fig. 1 ist eine in an sich bekannter Weise ausgebildete Vorrichtung zum Verbinden eines, insbesondere im Bereich des Oberschenkelhalses, gebrochenen Knochens bzw. Laschenschraube 10 mit einer Trochanterabstützplatte 20 kombiniert. Die Vorrichtung 10 besteht aus einer Knochenbefestigungslasche 11 mit einer Anzahl von in dieser ausgebildeten Durchbrechungen 12 zum Hindurchführen von Befestigungsschrauben, wenn die Vorrichtung 10 mit ihrer Knochenbefestigungslasche 11 an einem Knochen befestigt werden soll. Einendseitig trägt die Knochenbefestigungslasche 11 eine Hülse, ein zylindrisches Rohr od.dgl. 13, die zur Aufnahme und Führung eines in der Zeichnung nicht dargestellten Knochennagels oder einer Knochenschraube dient, wobei die Hülse 13 in einem Winkel zu der Knochenbefestigungslasche 11 steht, wie dies in Fig. 1 und 16 dargtestellt ist. Diese Vorrichtung 10 mit ihrer Knochenbefestigungslasche 11 und der Hülse 13 ist gemäß Fig. 1 und 16 mit der Trochanterabstützplatte 20 kombiniert, die ein für sich eigenes Bauelement in Form eines ganz speziell ausgebildeten Formkörpers darstellt.

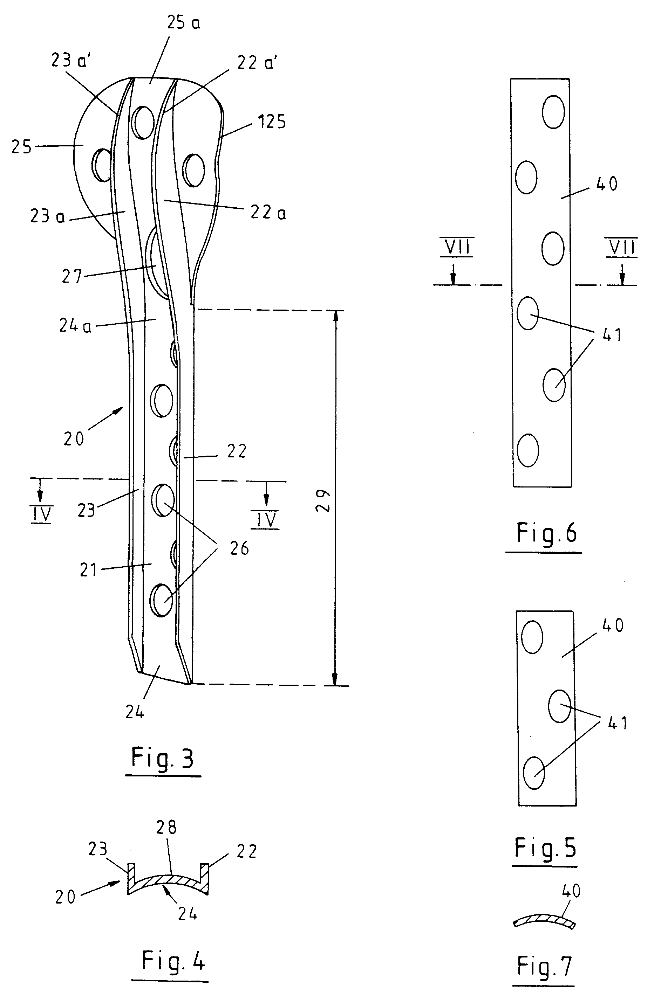

Diese Trochanterabstützplatte 20 besteht aus einem Profilkörper 21 mit einem geraden oder einem U-förmigen Querschnitt (Fig. 3 und 4). Die beiden, den Profilkörper 21 bildenden Schenkel sind mit 22,23 und der die beiden Schenkel 22,23 miteinander verbindende Steg ist mit 24 bezeichnet. Dieser Profilkörper 21 weist einen laschenförmigen Abschnitt 29 auf, der von den Schenkeln 22,23 und dem Steg 24 gebildet wird. Dieser laschenförmige Abschnitt 29 des Profilkörpers 21 entspricht in etwa der Form und Ausgestaltung der Knochenbefestigungslasche 11 der Vorrichtung 10.

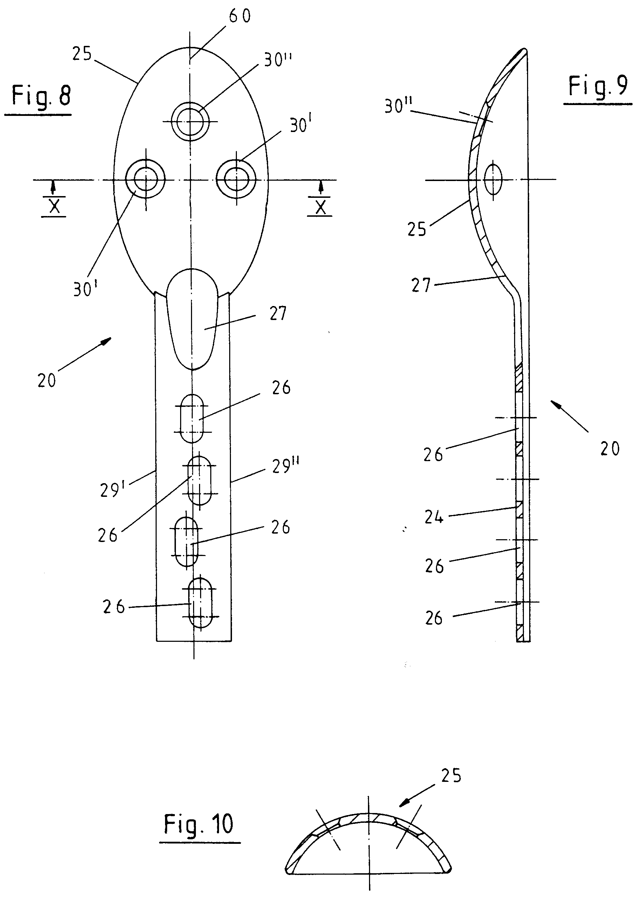

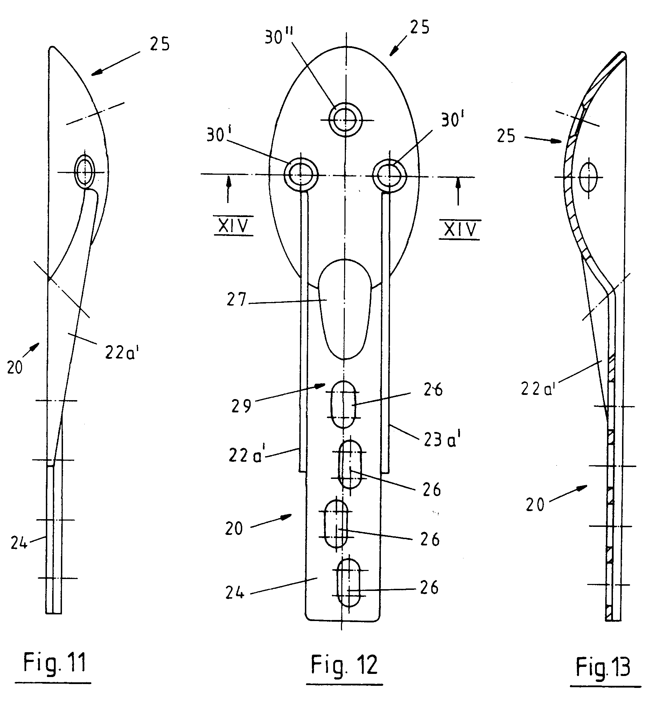

An dem Ende 24a des Profilkörpersteges 24 bzw. des laschenförmigen Abschnittes 29 ist an den Steg 24 ein plattenförmiger Abschnitt 25 angeformt, der sich kopfartig erweitert bzw. löffelartig ausgebildet ist und die Abstützfläche für den Trochanter bildet. Dieser plattenförmige Abschnitt 25 mit seiner Abstützfläche für den Trochanter bildet die eigentliche Trochanterabstützplatte. Dieser plattenförmige und die Abstützfläche für den Trochanter bildende Abschnitt 25 ist mit einer Anzahl von Durchbrechungen 30 versehen, die zur Aufnahme und zum Hindurchführen von Befestigungsnägeln oder -schrauben dienen (Fig.2). Bei den in Fig. 8 und 12 dargestellten Ausführungsbeispielen weist die Trochanterabstützplatte 20 drei Durchbrechungen 30',30'' auf, von denen die beiden Durchbrechungen 30' nebeneinanderliegend und vorzugsweise ovalförmig sind, während die dritte Durchbrechung 30'' im oberen Bereich der Trochanterabstützplatte 20 vorgesehen und kreisförmig ausgebildet ist.

Der Steg 24 bzw. der laschenförmige Abschnitt 29 des Profilkörpers 21 der Trochanterabstützplatte 20 weist in seinem Querschnitt eine kreisbogenförmige Auswölbung 28 auf, die in etwa der Knochenform angepaßt und die den beiden Profilkörperschenkeln 22,23 zugekehrt ist (Fig.4). Die Form und die Abmessungen dieser Auswölbung 28 entsprechen in etwa einem Abschnit der Auswölbung des Knochens, damit nach Möglichkeit eine vollflächige Anlage des Profilkörpers 21 mit seinem laschenförmigen Abschnitt 29 an einem Knochen gewährleistet ist.

Des weiteren weist der Profilkörper 21 in seinem Steg 24 und insbesondere in seinem laschenförmigen Abschnitt 29 eine Anzahl von Befestigungsdurchbrechungen 26 auf, die beim Einsetzen der Knochenbefestigungslasche 11 der Vorrichtung 10 in den Profilkörper 21 der Trochanterabstützplatte 20 mit den Befestigungsdurchbrechungen 12 in der Knochenbefestigungslasche 11 der Vorrichtung 10 zur Deckung bringbar sind, so daß eine gleichzeitige Befestigung der Vorrichtung 10 vermittels ihrer Knochenbefestigungslasche 11 und der Trochanterabstützplatte 20 möglich ist. Die Befestigungsdurchbrechungen 26 in dem Steg 24 bzw. dem laschenförmigen Abschnitt 29 des Profilkörpers 21 der Trochanterabstützplatte 20 erstrecken sich bis in den Bereich des plattenförmigen Abschnittes 25, der die Abstützfläche für den Trochanter bildet (Fig. 2 und 3).

Des weiteren erstrecken sich die Schenkel 22,23 des Profilkörpers 21, wie in Fig. 3 dargestellt, bis in den Endbereich, der bei 25a angedeutet ist, des plattenförmigen, die Abstützfläche für den Trochanter bildenden Abschnittes 25, wobei die im Bereich des plattenförmigen Abschnittes 25 verlängerten Abschnitte der beiden Schenkel 22,23 des Profilkörpers 21 bei 22a,23a angedeutet sind. Diese verlängerten Abschnitte 22a,23a der Schenkel 22,23 des Profilkörpers 21 der Trochanterabstützplatte 20 verjüngen sich zu ihren Enden 22a',23a' konisch. Außerdem sind die verlängerten Abschnitte 22a,23a der beiden Schenkel 22,23 im Bereich des plattenförmigen Abschnittes 25 dessen Formgebung angepaßt (Fig.3).

Neben den Befestigungsdurchbrechungen 26 weist der Steg 24 bzw. der laschenförmige Abschnitt 29 des Profilkörpers 21 der Trochanterabstützplatte 20 im übergangsbereich zum plattenförmigen Abschnitt 25 eine ovaläre Aussparung bzw. Durchbrechung 27 auf, die zum Hindurchführen der an der Knochenbefestigungslasche 11 der Vorrichtung 10 angeformten Hülse 13 dient und so bemessen ist, daß auch Vorrichtungen 10 in Verbindung mit der Trochanterabstützplatte 20 verwendet werden können, bei denen unterschiedliche Winkelstellungen zwischen der Hülse 13 und der Knochenbefestigungslasche 11 gegeben sind. Durch diese ovaläre Aussparung bzw. Durchbrechung 27 wird beim Einsetzen der Vorrichtung 10 in die Trochanterabstützplatte 20 die Hülse 13 bzw. die Tragschraube der Vorrichtung 10 hindurchgeführt. Die ovaläre Aussparung bzw. Durchbrechung 27 ist daher so bemessen, daß Laschen aller Winkelgrößen in Bezug auf ihre Hülse 13 verwendet werden können.

Die Abmessungen des den Trochanter abstützenden Plattenteils 25 der Trochanterabstützplatte 20 können beliebig gewählt sein. Es besteht hiernach die Möglichkeit, eine Anzahl von Trochanterabstützplatten 20 vorrätig zu halten, bei denen der den Trochanter abstützende Plattenteil bzw. plattenförmige Abschnitt 25 verschiedene Größen, also verschiedene Abmessungen aufweisen kann, so daß immer eine maximale Anpassungsfähigkeit an anatomische Gegebenheiten gewährleistet ist, wenn aus einer Vielzahl von vorrätig gehaltenen Trochanterabstützplatten 20 dann diejenige Trochanterabstützplatte zur Anwendung gelangt, deren plattenförmiger und die Abstützfläche für den Trochanter bildende Abschnitt 25 in seinen Abmessungen den Abmessungen des jeweiligen Trochanters entspricht, an dem die Trochanterabstützplatte 20 in Kombination mit der Vorrichtung 10 zur Anlage gebracht werden soll. Der Vorteil gegenüber den bekannten, mit einer fest angeformten Trochanterabstützplatte versehenen Vorrichtungen besteht hier darin, daß die in ihrer Herstellung mit hohen Kosten verbundenen Vorrichtungen 10 nicht in einem sehr umfassenden Sortiment vorliegen müssen, sondern daß der Chirurg mit einer geringen Anzahl von Vorrichtungen 10 auskommen kann, wenn ihm dafür eine größere Anzahl von Trochanterabstützplatten 20 mit unterschiedlichen Formgebungen und Anpassungen des plattenförmigen und die Abstützfläche des Trochanters bildenden Abschnittes 25 zur Verfügung steht, um eben diese Trochanterabstützplatte 20 dann mit der entsprechenden Vorrichtung 10 zu kombinieren.

Die Trochanterabstützplatte 20 besteht aus hochwertigem Material, das dem Material entspricht, aus dem die bekannten Vorrichtungen hergestellt werden. Dabei handelt es sich um körperfreundliche Materialien, wobei auch geeignete Kunststoffe zur Anwendung gelangen können, wenn diese den jeweiligen Erfordernissen entsprechen,die an eine derartige Trochanterabstützplatte 20 gestellt werden.

Die Abmessungen des Profilkörpers 21 der Trochanterabstützplatte 20 im Bereich seines laschenförmigen Abschnittes 29 ist dergestalt, daß die Knochenbefestigungslasche 11 der Vorrichtung 10 zwischen den beiden Profilschenkeln 22,23 liegend auf dem Profilschenkelsteg 24 zur Anlage bringbar ist.

Um den laschenförmigen Abschnitt 29 bzw. den Steg 24 des Profilkörpers 21 der Trochanterabstützplatte 20 verlängern zu können, sind gemäß Fig. 5 und 6 plattenförmige Verlängerungselemente mit unterschiedlichen Längen verwendbar. Fig. 5 zeigt ein derartiges Verlängerungselement 40 mit einer kleinen Länge, wohingegen das Verlängerungselement gemäß Fig. 6 eine größere Länge aufweist. Jedes Verlängerungselement 40 ist Platten- und streifenförmig ausgebildet und entspricht in einer Breite in etwa dem Steg 24 des Profilkörpers 21 der Trochanterabstützplatte 20. Außerdem ist das Verlängerungselement mit Befestigungsdurchbrechungen 41 versehen. Zur Verlängerung des laschenförmigen Abschnittes 29 der Trochanterabstützplatte 20 wird ein derartiges Verlängerungselement 40 auf den Steg 24 gelegt und zwischen den beiden Profilkörperschenkeln 22,23 so eingeschoben, daß die Befestigungsdurchbrechungen 41 des Verlängerungselementes 40 deckungsgleich mit den Befestigungsdurchbrechungen 26 im laschenförmigen Abschnitt 29 bzw. des Steges 24 des Profilkörpers 21 der Trochanterabstützplatte 20 zu liegen kommen. Es besteht darüber hinaus auch die Möglichkeit, im Bereich der Profilkörperschenkel 22,23 oder auf dem Steg 24 in der Zeichnung nicht dargestellte Führungen auszubilden, um ein derartiges plattenförmiges Verlängerungselement 40 einschieben und halten zu können. So besteht u.a. die Möglichkeit, in den einander gegenüberliegenden Wandflächen der beiden Profilkörperschenkel 22,23 benachbart zum Steg 24 bzw. zum laschenförmigen Abschnitt 29 Führungsnuten anzuordnen, die dann zur Aufnahme eines Verlängerungselementes 40 dienen, welches lediglich dann in diese Führungsnuten eingeschoben wird. Wie Fig. 7 zeigt, weist auch das plattenförmige Verlängerungselement 40 ein dem Wölbungsprofil des Steges 24 des Profilkörpers 21 der Trochanterabstützplatte 20 entsprechendes Kreisbogenprofil auf. Das Verlängerungselement 40 besteht aus den gleichen Materialien, aus denen die Vorrichtung 10 und die Trochanterabstützplatte 20 gefertigt sind.

Die in Fig. 2 mit A' und B' angegebenen Bereiche deuten die Varibilität der Abmessungen der Trochanterabstützfläche an.

Bei der in Fig. 8 bis 11 gezeigten Ausführungsform ist die Abstützfläche der Trochanterabstützplatte 20 löffelartig ausgebildet und ist an ihrem rückwärtigen Bereich mit in den laschenförmigen Steg 29 übergehenden Versteifungsstegen 22a',23a' versehen, die den verlängerten Abschnitten 22a,23a der Ausführungsform gemäß Fig. 2 entsprechen und die zu beiden Seiten der Längskanten 29', 29'' des laschenförmigen Steges 29 verlaufen (Fig. 12,13,15). Diese beiden Versteifungsstege 22a,23a gehen in die Rundung der löffelartigen Abstützfläche der Trochanterabstützplatte 20 über und erstrecken sich etwa bis an die beiden Durchbrechungen 30' in der löffelartigen Abstützfläche.

Auch bei dieser Ausführungsform weist die Trochanterabstützplatte 20 im Übergangsbereich des laschenförmigen Abschnittes 29 zum plattenförmigen Abschnitt 25 eine ovaläre Durchbrechung 27 auf, die zum Hindurchführen der an der Knochenbefestigungslasche 11 angeformten Hülse 15 dient (Fig.12,1 und 16). Die Durchbrechungen 26 in dem laschenförmigen Steg 29 sind zueinander versetzt angeordnet, wobei eine Anordnung der Durchbrechungen 26 wie in Fig. 8 dargestellt besonders vorteilhaft ist, nach der die dem plattenförmigen Abschnitt 25 zugekehrte Durchbrechung 26 auf der mittigen Längsachse 60 liegt, während die anderen Durchbrechungen 26 wechselseitig zur Längsachse 60 angeordnet sind (Fig.8).

Bei der in Fig. 12 bis 14 gezeigten Ausführungsform einer Trochanterabstützplatte 20 sind an dem laschenförmigen Abschnitt 29 bzw. an dessen Steg 24 die sich bei der Trochanterabstützplatte 20 gem. Fig. 1 und 2 über die gesamte Länge des Steges 24 bis in den Bereich des plattenförmigen, löffelartig ausgebildeten Abschnittes 25 erstreckenden Schenkel 22,23 nur abschnittsweise ausgebildet und erstrecken sich nur über den Verbindungsbereich des laschenförmigen Abschnittes 29 bis in den Abschnitt 25 bis unterhalb der beiden unteren Durchbrechungen 30 in dem Abschnitt 25, so daß nur der übergangsbereich versteift und stabilisiert ist und außerdem eine ausreichenmde Führung für die Knochenbefestigungslasche 11 gegeben ist.

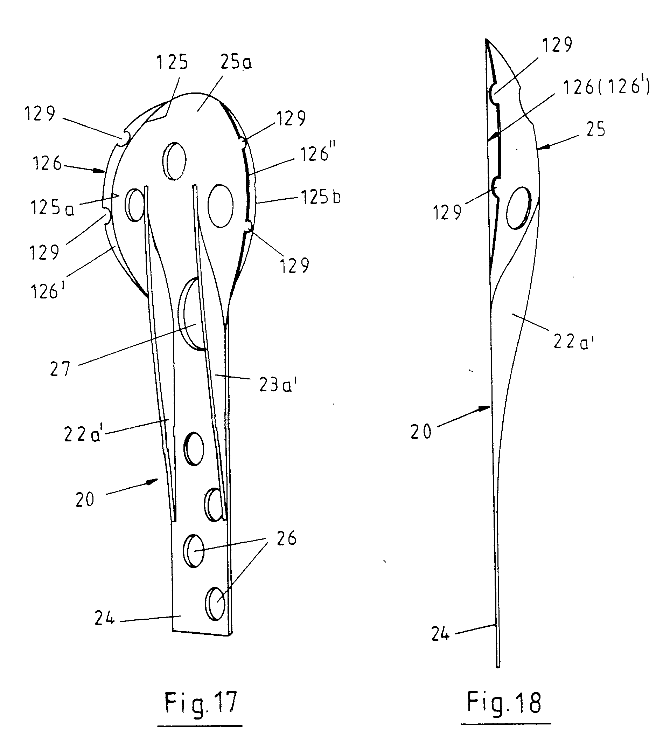

In den Fällen, in denen im Bereich des Trochanter major eine derartige Trümmerung vorhanden ist, daß die knöcherne Substanz nicht mehr schraubfähig ist, ist eine Anbindung und Fixierung vermittels Cerclage-Drähte erforderlich. Hierzu weist die Trochanterabstützplatte 20 eine Verbindungseinrichtung 70 auf, die ein Fixieren eines Cerclage-Drahtes oder mehrerer Cerclage-Drähte an der Trochanterabstützplatte 20 ermöglicht. Diese Verbindungseinrichtung 70 besteht aus einer Abwinklung des umlaufenden Randes 125 des plattenförmigen und löffelartig ausgebildeten Abschnittes 25 der Trochanterabstützplatte 20 in deren rückwärtigen Bereich. Dieser abgewinkelte, umlaufende Abschnitt 126 ist im wesentlichen in den mittigen Seitenbereichen 125a,125b des umlaufenden Randes 125 des Abschnittes 25 ausgebildet und erstreckt sich somit zu beiden Seiten des Abschnittes 25 der Trochanterabstützplatte 20, so daß zwei Einzelabschnitte 126',126'' ausgebildet sind. Die Enden der beiden Einzelabschnitte 126',126'' verjüngen sich konisch zu beiden Seiten (Fig.17).

Zur Fixierung eines in der Zeichnung nicht dargestellten Cerclage-Drahtes weist der abgewinkelte Abschnitt 126 bzw. seine Einzelabschnitte 126',126'' eine Anzahl von schlitzförmigen Ausnehmungen 129 auf.

## Patentansprüche

1. Vorrichtung zum Verbinden eines, insbesondere im Bereich des Oberschenkelhalses, gebrochenen Knochens, bestehend aus einer mit dem Knochen durch eine Schraubverbindung verbindbaren, parallel zur Knochenlängsachse verlaufenden Knochenbefestigungslasche (11) mit Befestigungsdurchbrechungen (26), mit einer abgewinkelten Hülse (13) zur Aufnahme einer Knochenschraube oder eines Knochennagels, sowie einer Trochanterabstützplatte (20), wobei der Steg (24) eines laschenförmigen Abschnittes (29) der Trochanterabstützplatte (20) einendseitig (24a) in einen sich erweiternden plattenförmigen Abschnitt (25) als Trochanterabstützfläche übergeht und beidseitig je einen sich in Steglängsrichtung erstreckenden Versteifungsschenkel (22,23) aufweist, der sich über eine Teillänge des Steges (24) oder über die gesamte Länge des Steges (24) bis in einen Teil oder bis in den Endbereich (25a) des plattenförmigen Abschnittes (25) erstreckt und sich zu dem Endbereich (25a) hin konisch verjüngt, wobei der plattenförmige Abschnitt (25) als Trochanterabstützfläche nach cranial über die Knochenbefestigungslasche (11) hinausragt, dadurch gekennzeichnet, daß die Trochanterabstützplatte (20) lösbar mit der Knochenbefestigungslasche (11) verbunden ist, und der laschenförmige Abschnitt (29) in etwa der Knochenbefestigungslasche (11) entsprechende Abmessungen aufweist, daß die Knochenbefestigungslasche (11) der Vorrichtung (10) zwischen den beiden Schenkeln (22,23) angeordnet und an dem Steg (24) zur Anlage gebracht ist, wobei der Steg (24) eine Anzahl von mit den Befestigungsdurchbrechungen (12) der Knochenbefestigungslasche (11) deckungsgleichen Befestigungsdurchbrechungen (26) und eine ovaläre Aussparung bzw. Durchbrechung (27) zum Hindurchführen der Knochenschraube oder Knochenaufnahmehülse (13) aufweist.

2. Vorrichtung nach Anspruch 1, dadurch gekennzeichnet, daß die Trochanterabstützplatte (20) aus einem einen U-förmigen Querschnitt aufweisenden Profilkörper (21) besteht.

3. Vorrichtung nach einem der Ansprüche 1 oder 2, dadurch gekennzeichnet, daß der Steg (24) des Profilkörpers (21) der Trochanterstützplatte (20) eine den Profilkörperschenkeln (22,23) zugekehrte kreisbogenförmigs Auswölbung (28) aufweist.

4. Vorrichtung nach einem der Ansprüche 1 bis 3, dadurch gekennzeichnet, daß die ovaläre Aussparung bzw. Durchbrechung (27) in dem Steg (24) des Profilkörpers (21) der Trochanterabstützplatte (20) den verschiedenen Winkelgrößen der Vorrichtung (10) angepaßt ist.

5. Vorrichtung nach einem der Ansprüche 1 bis 4, dadurch gekennzeichnet, daß der Profilkörper (21) der Trochanterabstützplatte (20) mittels aufsteckbarer Profilkörperteile verlängerbar ausgebildet ist.

6. Vorrichtung nach einem der Ansprüche 1 bis 5, dadurch gekennzeichnet, daß der umlaufende Rand (125) des löffelartigen Abschnittes (25) der Trochanterabstützplatte (20) in deren rückwärtigen Bereich abgewinkelt ist und daß der abgewinkelte Randabschnitt (126) eine Anzahl von Cerclage-Draht-Fixierungsausnehmungen (129) aufweist, wobei der abgewinkelte Randabschnitt (126) im oberen Endbereich (25a) des Abschnittes (25) der Trochanterabstützplatte (20) und in den Übergangsbereichen der Stege (22,23, 22a',23a') in dem Abschnitt (25) sich konisch verjüngend verlaufend ausgebildet ist.

7. Vorrichtung nach einem der Ansprüche 1 bis 5, dadurch gekennzeichnet, daß der umlaufende Rand (125) des löffelartigen Abschnittes (25) der Trochanterabstützplatte (20) zu beiden Seiten der Trochanterabstützplatte (20) in deren rückwärtigen Bereich unter Ausbildung von zwei Randabschnitten (126',126'') abgewinkelt ist, und daß jeder Randabschnitt (126', 126'') eine Anzahl von Cerclage-Draht-Fixierungsausnehmungen (129) aufweist, wobei jeder Randabschnitt (126',126'') in seinen beiden Endbereichen konisch sich verjüngend auslaufend ausgebildet ist.

8. Vorrichtung nach einem der Ansprüche 6 oder 7, dadurch gekennzeichnet, daß der abgewinkelte Randabschnitt (126) auf jeder Seite je zwei Cerclage-Draht-Fixierungsausnehmungen (129) bzw. jeder der beiden seitlichen Randabschnitte (126',126'') des Abschnittes (25) der Trochanterabstützplatte (20) zwei Cerclage-Draht-Fixierungsausnehmungen (129) aufweist.

## Claims

1. Device for the fixation of a broken bone, in particular in the region of the femoral neck, comprising a bone fixation plate (11) proceeding parallelly to the longitudinal axis of the bone with attachment perforations (26), with an angled sleeve (13) for receiving a bone screw or fracture pin, as well as a trochanter supporting plate (20), in which the web (24) of a tongue-shaped section (29) of the trochanter supporting plate (20), on one end (24a), passes into an expanding plate-shaped section (25) in the form of a trochanter supporting region and, on both sides, is provided with a bracing leg (22,23) extending in the longitudinal direction of the web, which extends across a part of the length of the web (24) or over the entire length of the web (24) as far as into a part or as far as into the terminal area (25a) of the plate-shaped section (25) and, toward the terminal area (25a), is conically tapered, while the plate-shaped section (25) projects cranially above the bone fixation plate (11) as a trochanter supporting surface area,
**characterized in that**
the trochanter supporting plate (20) is detachably connected with the bone fixation plate (11) and in that the tongue- shaped section (29) possesses dimensions that correspond approximately to those of the bone fixation plate (11), in that the boner fixation plate (11) of the device (10) is disposed between the two legs (22,23) and are caused to rest upon the web (24) while the web (24) is provided with a plurality of attachment perforations (12) of the bone fixation plate (11) and possesses an oval cutout or perforation (27) for the bone screw or bone receiving sleeve (13) to be passed through.

2. Device according to Claim 1,
**characterized in that**
the trochanter supporting plate (20) is comprised of a sectional member (21) possessing a U-shaped cross-section.

3. Device according to either Claim 1 or 2,
**characterized in that**
the web (24) of the sectional member (21) of the trochanter supporting plate (20) possesses an arcuately configured outward bulge (28) facing the legs (22,23) of the sectional member.

4. Device according to any of Claims 1 to 3,
**characterized in that**
the oval cutout or perforation (27) in the web (24) of the sectional member (21) of the trochanter supporting plate (20) is adapted to the different angular sizes of the device (10).

5. Device according to any of Claims 1 to 4,
**characterized in that**
the sectional member (21) of the trochanter supporting plate (20) is constructed so as to be extensible with the aid of attachable sectional member portions.

6. Device according to any of Claims 1 to 5,
**characterized in that**
the circumferential edge (125) of the spoon-shaped section (25) of the trochanter supporting plate (20) is angled in its rearward area, and in that the angled marginal section (126) isd provided with a plurality of cerclage wire fixation notches (129), while the angled marginal section (126), within the terminal area (25a) of the section (25) of the trochanter supporting plate (20) as well as in the transitional areas of the webs (22,23,22a',23a') within the section (25) is constructed so as to be conically tapering.

7. Device according to any of Claims 1 to 5,
**characterized in that**
the circumferential edge (125) of the spoon-shaped section (25) of the trochanter supporting plate (20), on both sides of the trochanter supporting plate (20) within its rearward area is angled while forming two marginal sections (126',126''), and in that each marginal section (126'126'') possesses a plurality of cerclage wire fixation notches (129), while each marginal section (126',126''), within the terminal areas, is constructed so as to proceed conically tapering.

8. Device according to either Claim 6 or 7,
**characterized in that**
the angled marginal section (126) possesses on each side two cerclage wire fixation notches (129) each or in that each of the two lateral marginal sections (126',126'') of the section (25) of the trochanter supporting plate (20) is provided with two cerclage wire fixation notches (29).

## Revendications

1. Dispositif pour fixer un os fracturé, en particulier dans la région du col du fémur, constitué par un collier de fixation d'os (11), qui peut être relié à l'os par un assemblage à vis et qui est parallèle à l'axe longitudinal de l'os, avec des découpures de fixation (26), avec une douille coudée (13) pour loger une vis ou une broche pour os, ainsi que par une plaque d'appui de trochanter (20), la traverse (24) d'une section en forme de collier (29) de la plaque d'appui de trochanter (20) se fondant à l'une des extrémités (24a) en une section en forme de plaque (25) qui s'élargit comme surface d'appui de trochanter et présentant sur chacun des deux côtés un montant de renfort (22, 23) qui s'étend dans le sens longitudinal de la traverse qui s'étend sur une longueur partielle de la traverse (24) ou sur toute la longueur de la traverse (24) jusque dans une partie ou jusque dans la zone d'extrémité (25a) de la section en forme de plaque (25) et qui s'effile de manière conique vers la zone d'extrémité (25a), la section en forme de plaque (25) comme surface d'appui du trochanter faisant saillie vers le haut au-dessus du collier de fixation de l'os (11), **caractérisé en ce** que la plaque d'appui de trochanter (20) est reliée de manière amovible au collier de fixation de l'os (11) et la section en forme de collier (29) présente approximativement des dimensions qui correspondent au collier de fixation de l'os (11), que le collier de fixation de l'os (11) du dispositif (10) est placé entre les deux montants (22, 23) et vient s'appliquer contre la traverse (24), la traverse (24) présentant un certain nombre de découpures de fixation (26) qui coïncident avec les découpures de fixation (12) du collier de fixation de l'os (11) et un évidement ou une découpure (27) ovale pour faire passer la vis pour l'os ou la douille de logement de l'os (13).

2. Dispositif selon la revendication 1, **caractérisé en ce** que la plaque d'appui de trochanter (20) est constituée par un corps profilé (21) qui présente une section en forme de U.

3. Dispositif selon l'une des revendications 1 ou 2, **caractérisé en ce** que la traverse (24) du corps profilé (21) de la plaque d'appui de trochanter (20) présente une voussure (28) en forme d'arc de cercle tournée vers les montants du corps profilé (22, 23).

4. Dispositif selon l'une des revendications 1 à 3, **caractérisé en ce** que l'évidement ou la découpure ovale (27) dans la traverse (24) du corps profilé (21) de la plaque d'appui de trochanter (20) est adapté aux différentes grandeurs d'angle du dispositif (10).

5. Dispositif selon l'une des revendications 1 à 4, **caractérisé en ce** que le corps profilé (21) de la plaque d'appui de trochanter (20) est configurée en pouvant être prolongée au moyen de parties de corps profilé pouvant être emboîtées.

6. Dispositif selon l'une des revendications 1 à 5, **caractérisé en ce** que le bord périmétrique (125) de la section du type cuillère (25) de la plaque d'appui de trochanter (20) est coudé dans sa zone arrière et que la zone marginale coudée (126) présente un certain nombre d'évidements de fixation du fil de cerclage (129), la zone marginale coudée (126) étant formée en s'effilant de manière conique dans la zone d'extrémité supérieure (25a) de la section (25) de la plaque d'appui de trochanter (20) et dans les zones de transition des traverses (22, 23, 22a', 23a') dans la section (25).

7. Dispositif selon l'une des revendications 1 à 5, **caractérisé en ce** que le bord périmétrique (125) de la section du type cuillère (25) de la plaque d'appui de trochanter (20) est coudé des deux côtés de la plaque d'appui de trochanter (20) dans sa zone arrière en formant deux sections marginales (126', 126'') et que chaque section marginale (126', 126'') présente un certain nombre d'évidements de fixation du fil de cerclage (129), chaque section marginale (126', 126'') étant configurée en se terminant en s'effilant de manière conique dans ses deux zones d'extrémité.

8. Dispositif selon l'une des revendications 6 ou 7, **caractérisé en ce** que la section marginale coudée (126) présente de chaque côté respectivement deux évidements de fixation du fil de cerclage (129) ou chacune des zones marginales latérales (126', 126'') de la section (25) de la plaque d'appui du trochanter (20) présente deux évidements de fixation du fil de cerclage (129).
